# EUROPEAN PATENT SPECIFICATION

(11) **EP 3 858 255 B1**
(45) Date of publication and mention of the grant of the patent: **22.07.2026**
(21) Application number: 21164430.7
(22) Date of filing: 26.05.2016
(51) Int. Cl.: A61B 17/00, A61M 1/00

(54) **A FISTULA TREATMENT DEVICE**
FISTELBEHANDLUNGSVORRICHTUNG
DISPOSITIF DE TRAITEMENT D'UNE FISTULE

(30) Priority: 28.05.2015 EP 15169640
(43) Date of publication of application: 04.08.2021
(62) Divisional of application: 19186010.5
(73) Proprietor: National University of Ireland Galway, Galway (IE)
(72) Inventor: BAMBURY, Eoin, Galway, County Galway H91 NNY6 (IE); ZILVERSMIT, Moshe, Galway, County Galway H91 NNY6 (IE)
(74) Representative: Weldon O'Brien Ltd.

(56) References cited:
- WO-A1-96/03925
- WO-A1-97/07744
- CN-A- 101 347 348
- US-A- 4 762 453
- US-A1- 2008 004 640

## Description

### Introduction

The invention relates to a device and a system for the treatment of perianal fistulas.

A perianal fistula is an artificial tunnel that, in the majority of cases, develops from an infection that begins within a blocked anal gland. If the infection cannot be cleared from the anal gland an abscess forms and the infection burrows though the sphincteric muscles and exits at the buttocks integument. Patients experience pain associated with the tract and associated abscesses and suffer faecal and blood discharge from the fistula tract. Perianal fistulas may also result from gastrointestinal diseases such as Crohn's disease, ulcerative colitis, colorectal cancers and their associated treatment and complications due to rectal fissures and trauma.

The global incidence of perianal fistula is 2 per 10,000 population. Over 100,000 fistula procedures are performed between the United States and Western Europe each year. Thirty percent of the procedures performed are reoperations due to treatment failure contributing to a significant preventable cost to the healthcare systems.

Given the inadequate treatment options and poor surgical outcomes there is a defined clinical need for a more effective perianal treatment device.

There is currently no single "gold standard" technique that a surgeon can perform to effectively cure a perianal fistula and not render the patient incontinent. A common fistula treatment is a fistulotomy procedure. A fistulotomy involves the dissection of the sphincteric muscles and the laying open of the fistula tract. Fistulotomies have a relatively high cure rate, however, this procedure results in a high risk of faecal incontinence.

From a patient's point of view, many are happy to assume the risk of incontinence in order to resolve the painful fistula tract. However, this is obviously not an ideal treatment pathway and for many patient population groups the secondary outcome is far from acceptable.

Another commonly used fistula treatment methodology is the use of a seton. Setons are used as a sphincter sparing technique and is simply a suture or vascular strap that is passed through the tract of the fistula and the rectum and tied in a loop. The seton maintains tract patency allowing the infection in the fistula tract to drain, help the tract constrict in length and may cure the tract. If the tract is not cured by the seton the physician can perform a fistulotomy. This approach of trying to preserve the sphincter with setons has been used for over 2500 years and is still the preferred method used by surgeons today.

In an effort to provide a non-destructive perianal fistula treatment various glues and plugs have been developed and introduced to colorectal surgeons in the past 20 years. However, these techniques are not very successful and their use is not widespread. Such glues which are injected into the fistula tract generally become brittle and are not able to occlude the tract for a long enough period to fully heal, faeces re-enter the tract resulting in abscess formation and refistulisation. Physicians often attempt to treat perianal fistulas with glues and plugs even though there may only be a 10 percent chance of effectiveness because it is a sphincter sparing technique and they can always resort to a seton and eventual fistulotomy if all fails.

Attempts have also been made to use plugs to occlude perianal fistula tracts. For example, US2005/004926A describes a plug-like fistula closure device with an attached flexible application string which also serves to evacuate liquids out of a fistula. However, generally the plugs fail because they become extruded from the tract, allowing faeces to enter the tract resulting in reinfection, abscess formation and refistulisation.

US4762453 describes a helical fastener having a retaining member with a top portion and an elongated hub extending downwardly from the top portion.

US2008004640 describes a spiral closure device which is adapted to be threaded into the vessel wall surrounding the opening in the bodily vessel.

CN101347348 describes a neoplasty screw nail.

WO9603925 describes a helical fastener having a high retentive surface area is provided and has a first end for enhancing penetration into tissue.

WO9707744 tissue anchor which is an open helix of biocompatible material having a slope.

There is therefore a need for an improved method and device for the treatment of perianal fistulas.

### Statements of Invention

According to the invention there is provided a perianal fistula treatment system according to claim 1. Various features of the system are set out in the dependent claims 2 to 12.

### Brief Description of the Drawings

The invention will be more clearly understood from the following description of an embodiment thereof, given by way of example only, with reference to the accompanying drawings, in which the implant coils of Figs. 11 to 23 are not within the scope of the invention:
Fig. 1 is a cross-sectional view of an anatomy and disease state addressed by the invention;
Fig. 2 is a cross-sectional view of the treatment device of the invention being delivered through the fistula tract;
Fig. 3 is cross-sectional view of the device at a point of apposition to the mucosal surface at the site of the internal opening of the fistula tract;
Fig. 4 is cross-sectional view of the device partially delivered through the mucosal surface and into the sphincter muscle complex;
Fig. 5 is cross-sectional view of the device fully delivered into the sphincter muscle complex and detached from the delivery mechanism;
Fig. 6 is a plan view illustrating the internal opening of a fistula tract at the mucosal surface;
Fig. 7 is a plan view illustrating the device located at the internal opening of a fistula tract in apposition with the mucosal surface;
Fig. 8 is a plan view illustrating the device partially delivered through the mucosal surface and beginning to gather tissue internally to the device resulting in narrowing of the fistula internal opening;
Fig. 9 is a plan view illustrating the device further delivered through the mucosal surface and into the sphincter muscle complex with further gathering of tissue internally to the device resulting in additional narrowing of the fistula internal opening;
Fig. 10 is a plan view illustrating the device completely delivered sub-mucosally and entirely into the sphincter muscle complex with tissue internal to the device resulting in complete closure of the fistula internal opening;
Fig. 11 is a front view of an embodiment of a perianal fistula treatment device;
Fig. 12 is a plan view of the device of Fig. 11;
Fig. 13 is a side view of the device of Fig. 11 and 12;
Fig. 14 is a front view of another fistula device with pointed tip;
Fig. 15 is a side view of the device of Fig. 14;
Fig. 16 is a plan view of the device of Figs. 14 and 15;
Fig. 17 is an isometric view of the device of Figs. 14 to 16;
Fig. 18 is an isometric view of a further fistula treatment device with pointed tip and centre feature;
Fig. 19 is a front view of the device of Fig. 18;
Fig. 20 is a side view of the device of Figs. 18 and 19;
Fig. 21 is a plan view of the device of Figs. 18 to 20;
Fig. 22 is an isometric view of a device with pointed tip and centre feature and seton;
Fig. 23 is an isometric view of a device with pointed tip and centre seton attachment feature with attached seton;
Fig. 24 is a cross-sectional view of a cross shape seton;
Fig. 25 is a cross-sectional view of an oval shape seton;
Fig. 26 is a cross-sectional view of a round shape seton;
Fig. 27 is a cross-sectional view of a round hollow shape seton;
Fig. 28 is an isometric view of a round hollow perforated embodiment of a seton;
Fig. 29 is a cross-sectional view of a hollow star shape seton;
Fig. 30 is a cross-sectional view of a star shape seton;
Fig. 31 is an isometric view of a star shape hollow perforated embodiment of a seton;
Fig. 32 is a cross-sectional view of a cross shape seton;
Fig. 33 is a cross-sectional view of a multi-braid embodiment of a seton;
Fig. 34 is an isometric view of an oval cross-section embodiment of the closure coil device;
Fig. 35 is an isometric view of a ribbon type tapered coil of a perianal fistula treatment device;
Fig. 36 is an isometric view of a triangular cross-section embodiment of a tapered coil;
Fig. 37 is an isometric view of a multi-faced cross-section embodiment of the tapered coil;
Fig. 38 is an isometric view of a hollow delivery mechanism of the tapered coil;
Fig. 39 is an isometric view of a solid coil to be delivered by the hollow delivery mechanism of Fig. 38;
Fig. 40 is an isometric view of the hollow delivery mechanism of Fig. 38 and the solid coil of Fig. 39 in place;
Fig. 41 is an isometric view of a solid core delivery mechanism with a hollow coil in place;
Fig. 42 is an isometric view of a rail support structure for delivering a mating coil;
Fig. 43 is an isometric view of a coil that mates with the support structure of Fig. 42;
Fig. 44 is an isometric view of the combination of a coil and rail type delivery mechanism;
Fig. 45 illustrates the component parts of the system;
Fig. 46 illustrates the delivery of the system through the fistula tract prior to deployment of the coil;
Fig. 47 illustrates the position of the coil prior to deployment;
Fig. 48 illustrates a ball type centre feature seton attachment mechanism;
Fig. 49 illustrates a hook type centre feature seton attachment mechanism;
Fig. 50 illustrates a cleat type centre feature seton attachment mechanism;
Fig. 51 illustrates a hollow type centre feature seton attachment mechanism;
Fig. 52 illustrates a hollow type centre feature seton attachment mechanism;
Fig. 53 illustrates a swage type centre feature seton attachment mechanism;
Fig. 54 illustrates a crimp type centre feature seton attachment mechanism;
Fig. 55 illustrates a thermal bond type centre feature seton attachment mechanism;
Fig. 56 illustrates a butt joint type centre feature seton attachment mechanism;
Fig. 57 is an isometric view of a coil with a partially straight section;
Fig. 58 and Fig. 59 are isometric views of a hollow coil delivery mechanism interfaced with a solid coil; and
Fig. 60 and Fig. 61 are isometric views of a solid coil delivery mechanism interfaced with a hollow coil.

### Detailed Description

The device is capable of one or more of the following:
- accommodating varied fistula tract physiology;
- occluding and sealing the internal opening of the tract;
- preventing faecal matter re-infecting the tract;
- preserving sphincteric function;
- enhancing fistula tract healing; and
- facilitating drainage during healing.

The perianal fistula treatment device ensures sparing of the sphincter, occluding of the fistula tract internal opening, and promotion of drainage and tissue healing.

The device consists of a head with anchoring and sealing mechanisms which is secured in the tissue tract and prevents re-infection of the wound. A tail section provides seton-like drainage and prevents re-abscessing due to premature closure of the skin site.

The anchoring and sealing mechanism of the device consists of a tapered coil. The coil geometry is designed to pull tissue together as is it deployed into the sphincter muscle complex, resulting in a strong anchor but also, importantly, an effective compressive seal preventing reinfection of the fistula tract and close tissue approximation to enhance tissue healing.

The perianal fistula treatment device preserves sphincteric and anatomical conditions and functions, prevents re-fistulisation, and improves healing time over the current treatment methods. The device consists of a tapered coil and a drainage seton. There may be a centering alignment feature. A delivery mechanism is also described. The coil may be led into the fistula tract by the drainage seton and centered into the tract by means of the centering feature. The larger diameter of the tapered coil is abutted against the tissue surface, surrounding the internal opening of the fistula tract with adequate margin. The delivery mechanism rotates the coil until it is just submucosal positioned. The coil closes the fistula internal opening by compressing the tract's surrounding tissue inwardly such that the tissue is brought within close approximation creating a seal impermeable to foreign materials and promoting tissue growth across the closely approximated fistula tract. The drainage seton provides a conduit to drain any abscess and remaining or newly formed exudate and fluids from the fistula tract throughout the time of the healing process. The centering feature insures the coil device is placed easily into the fistula tract and the outer coil is placed within the adequate margins surrounding the fistula tract and acts as a securing mechanism for the drainage seton.

The following numerals are used in the drawings:
1. Rectum
2. Anus
3. Internal Sphincter Muscle
4. External Sphincter Muscle
5. Dentate Line
6. Fistula Tract
7. Internal Opening
8. External Opening
9. Anal Verge
10. Levator Ani Muscle
11. Coil
12. Seton
13. Seton Distal End
14. Seton Attachment
15. Coil Distal Tip
16. Delivery Mechanism Member
17. Delivery Mechanism Interface
18. Delivery Mechanism
19. Internal Opening Mucosal Surface
20. Musocal Surface
21. Internal Opening
22. Compressed Sphincter Muscle Tissue
23. Coil
24. First End
25. Second End
26. Medial Portion
27. Coil Member
28. Pointed Tip
29. Delivery Mechanism Interface Feature
30. First End
31. Second End
32. Coil Member
33. First End
34. Second End
35. Pointed Tip
36. Centre Feature
37. Coil Member
38. Delivery Mechanism Interface Feature
39. Second End
40. Delivery Mechanism Interface Feature
41. First end
42. Pointed Tip
43. Coil Member
44. Centre Seton Feature
45. First End
46. Second End
47. Pointed Tip
48. Delivery Mechanism Interface Feature
49. Coil Member
50. Centre Feature
51. Seton Attachment Feature
52. Drainage Seton
53. Drainage Seton End
54. Cross Shape Seton Cross Section
55. Oval Seton Cross Section
56. Round Seton Cross Section
57. Outer Wall Hollow Round Seton Cross Section
58. Round Hollow Cross Section
59. Hollow Perforated Seton
60. First End
61. Second End
62. Hollow Centre Section
63. Perforation
64. Hollow Star Seton Cross Section
65. Solid Star Seton Cross Section
66. Hollow Perforated Star Seton
67. First End
68. Second End
69. Hollow Feature
70. Perforation
71. Cross Shape Seton Cross Section
72. Braided Seton Cross Section
73. Integument
200. Implant coil
201. Coil straight section
202. Coil tapered section
203. Transition from straight to tapered section
204. Hollow delivery assembly
205. Hollow delivery coil
206. Solid coil implant
207. Coil straight section
208. Coil tapered section
209. Interface
210. Solid delivery assembly
211. Hollow coil implant
212. Solid delivery coil
213. Coil straight section
214. Coil tapered section
220. Leading end
221. Trailing end

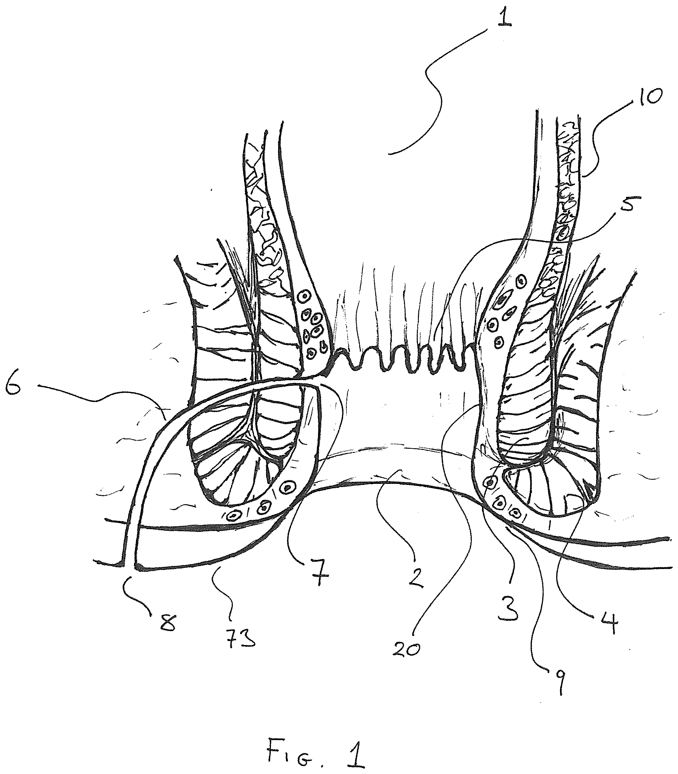

Fig. 1 illustrates an anal fistula tract 6, which is an abnormal connection between the rectum 1 and the integument of the buttocks 73. The internal opening 7 of the fistula is located at the mucosal surface 20 of the rectum 1. The fistula tract 6 may generally originate at the dentate line 5 and pass through the sphincteric muscle complex which consists of the internal sphincter muscle 3 and external sphincter muscle 4. The external opening 8 of the fistula tract 6 is located at the integument surface of the buttocks 73.

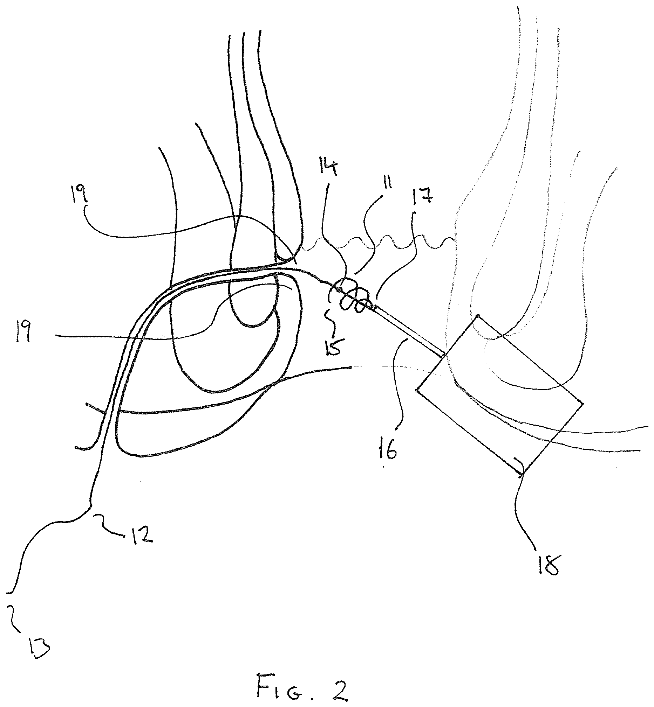

Fig. 2 shows the device consisting of a tapered coil 11, drainage seton 13, and delivery mechanism 18 being drawn through the fistula tract by means of tension applied to second end of drainage seton 13.

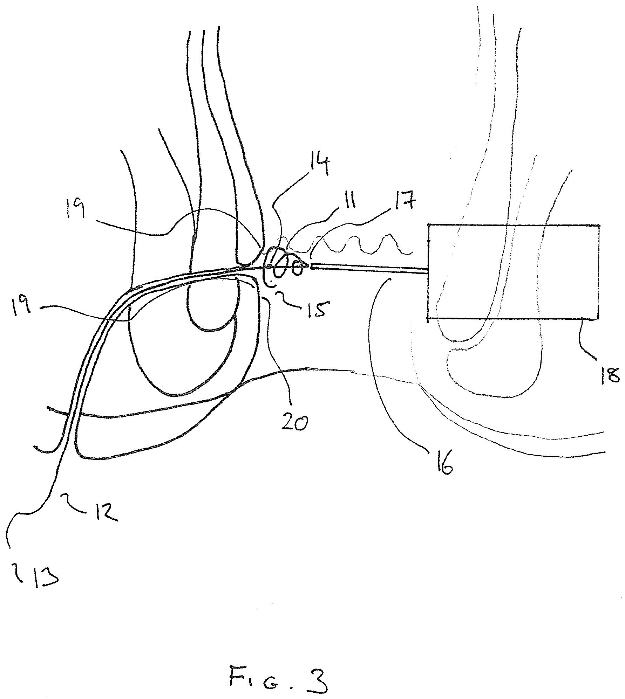

The tapered coil 11 is brought into apposition to the mucosal tissue wall of the rectum 20 as shown in Fig. 3. The coil is centered on the internal opening of the fistula tract 7 at the dentate line 5 via tension applied to the second end 13 of the drainage seton 12 and the support of the bioabsorbable mechanism 18 interface 17.

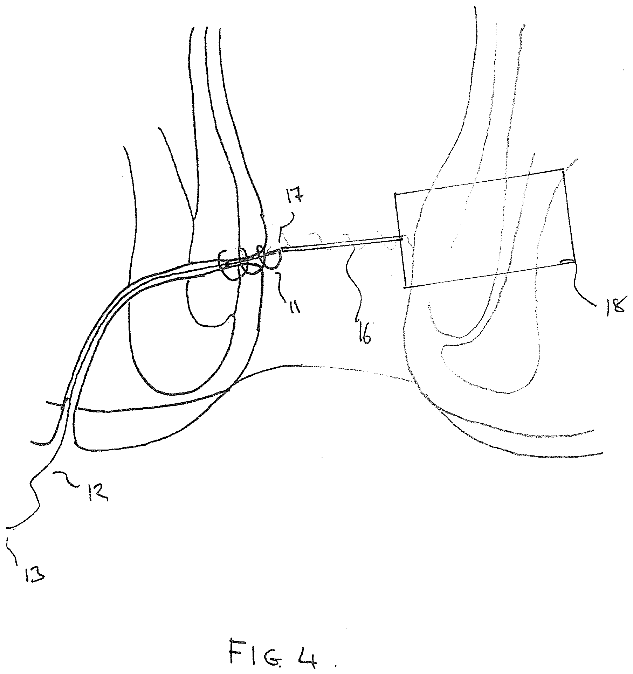

The driver mechanism 17 delivers the coil 11 through the mucosal lining 20 of the rectum 1 via rotatory or other means (Fig. 4). The distal tip 15 of the coil 11 punctures through the musocal lining surface 20 and engages initially with the internal sphincter muscle 3 surrounding the internal opening 7 of the fistula tract 6.

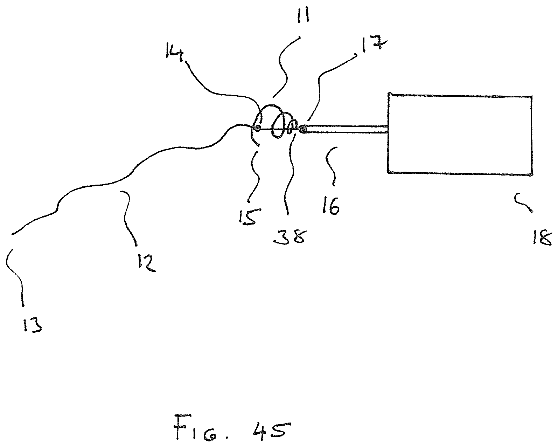

Fig. 45 illustrates the system component parts. The delivery mechanism interface feature 38 of the coil 11 is attached to the delivery mechanism member 16 of the delivery mechanism 18. The drainage seton 12 is attached to a coil centering feature 50 via the seton attachment feature 14.

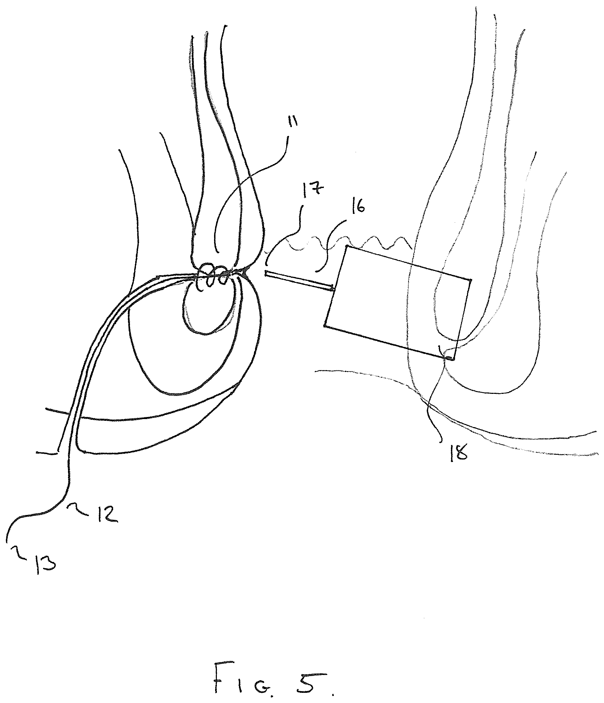

Fig. 5 illustrates the coil 11 completely delivered through and past the mucosal surface 20 and into the sphincter complex consisting of the internal 3 and external 4 sphincter muscles. The delivery mechanism interface 17 located distally on the delivery mechanism member 16 detaches from the tapered coil 11 and the delivery mechanism 18 is removed from the surgical field.

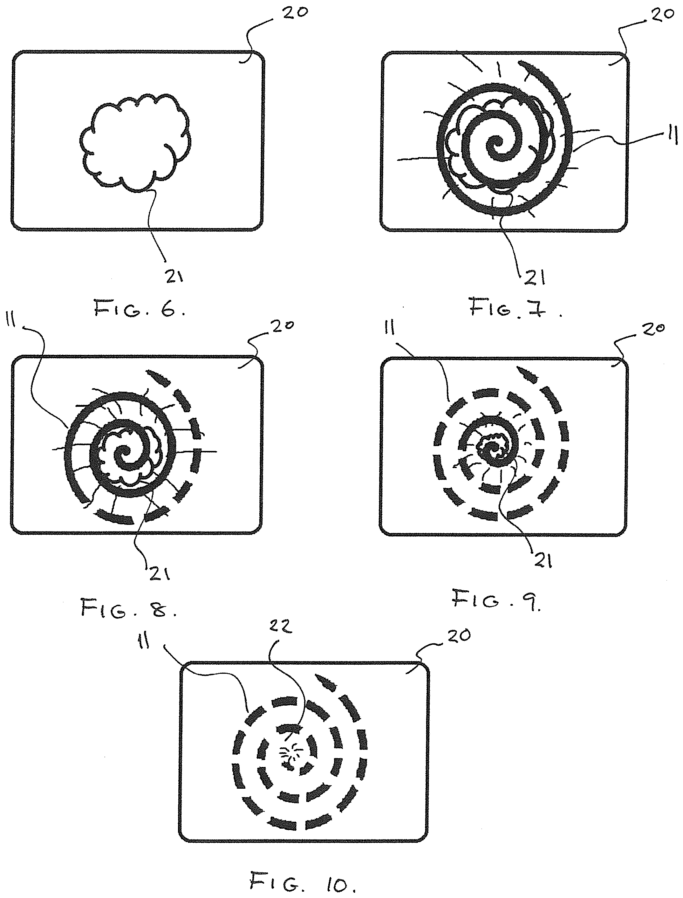

The mechanism of action of the delivery of the tapered coil results in sphincter muscle complex tissue being drawn into the centre of the coil 11 construct. The mechanism of action is illustrated in Figs. 6 - 10.

Fig. 6 illustrates a plan view of an internal opening 21 of a fistula tract 6 located at the surface of the musical lining of the rectum 20 at the dentate line 5. Fig. 7 shows the closure coil 11 being brought into apposition to the musical surface 20 and being centred on the fistula tract 7 internal opening 21. As the closure coil 11 is delivered through the mucosal surface 20 and into the internal sphincter muscle 3 the sphincter muscle tissue begins to be gathered into the centre of the closure coil 11. Fig. 9 illustrates further delivery of the closure coil 11 resulting in an increased mass of sphincter muscle tissue being gathered internally in the coil. Fig. 10 illustrated complete delivery of the closure coil 11 entirely past the mucosal surface 20 and completely into the sphincter muscle complex including the internal 3 and external 4 sphincter muscles. The complete delivery of the coil 11 results in closure of the internal opening 21 of the fistula tract 6 by mechanism of gathering and compression of sphincter muscle tissue. This mechanism as described allows both the sphincter muscle tissue to knit together, and the mucosal surface to remodel to cover the site of delivery over a period of time, eventually resulting in complete resolution of the sphincter muscle defect associated with the internal opening of the fistula tract.

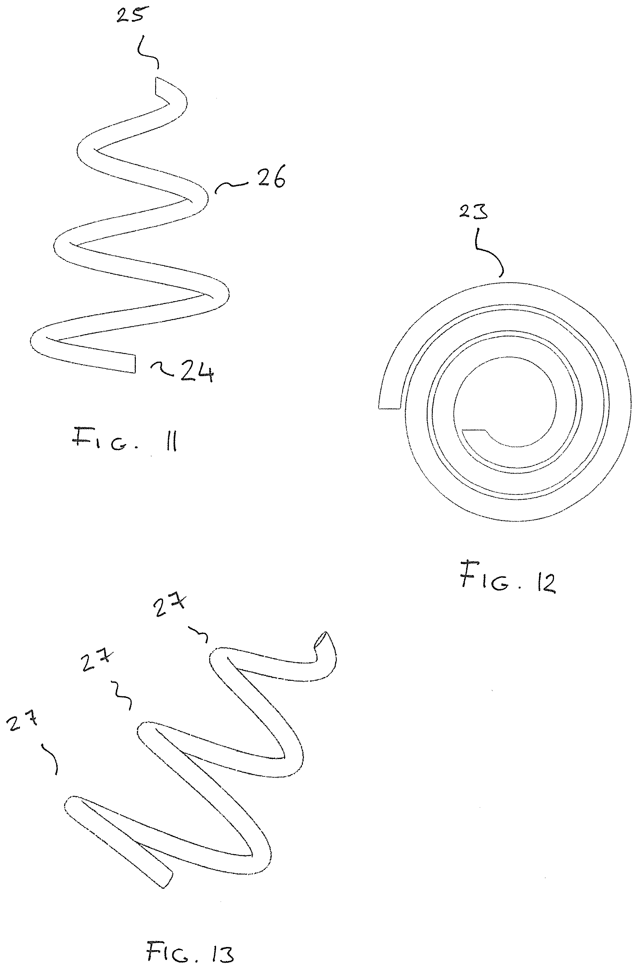

Referring to Figs. 11 to 13 there is illustrated a tapered coil 12 which has a leading end 24 and a trailing end 25. The coil is in this case conical and decreases in lateral extent between the leading and trailing ends.

The implant is a coiled body structure. The leading end of the implant is the largest coil and initially surrounds the tissue defect with appropriate margin. As the implant is advanced the leading end provides a large surface area to effectively anchor the implant. Each subsequent coil provides (adds to) the anchoring and compression function. The smallest coil towards the trailing end provides the highest amount of tissue compression. As the implant is turned into the tissue each coil further compresses the captured tissue toward the center of the tissue defect, thus effectively completely compressing the surrounding tissue inwardly. The close approximation of tissue allows for the tissue to heal together. This compression provides an effective seal against the pressures generated in the rectum and prevents entering of passing faeces into the fistula tract thus preventing re-infection. The smaller diameters of the implant coils retain the captured tissue from separating and prevents the breakdown of the healing process or foreign material from entering the tissue defect. This is a major advantage over sutures and suture based surgical techniques such as the advancement flap (dermal flap) and the LIFT procedures.

The compression ensures close approximation of tissue throughout the center of the implant. At the most proximal surface the close approximation of tissue provides support to the healing mucosal lining of the rectum over the implant and tissue defect. Thus the healing tissue is fully supported by the implant during the healing process and is capable of surviving pressures of 150mmHg and upwards of 200mmHg which can be generated in the rectum.

The coil is delivered submucosal (at a predetermined depth) below the surface of the mucosa. This is to ensure that there is a full mucosal seal at the rectal mucosa surface to provide for a bacterial seal barrier. With the implant just below the surface the tissue is drawn inwards for complete compression and supports the mucosa healing process.

As the implant is turned into the tissue the compression becomes greater along the depth of the coil (progressive compression) and the length of the tract captured internal of the implant is compressed completely. This close approximation of tissue aids in the healing process.

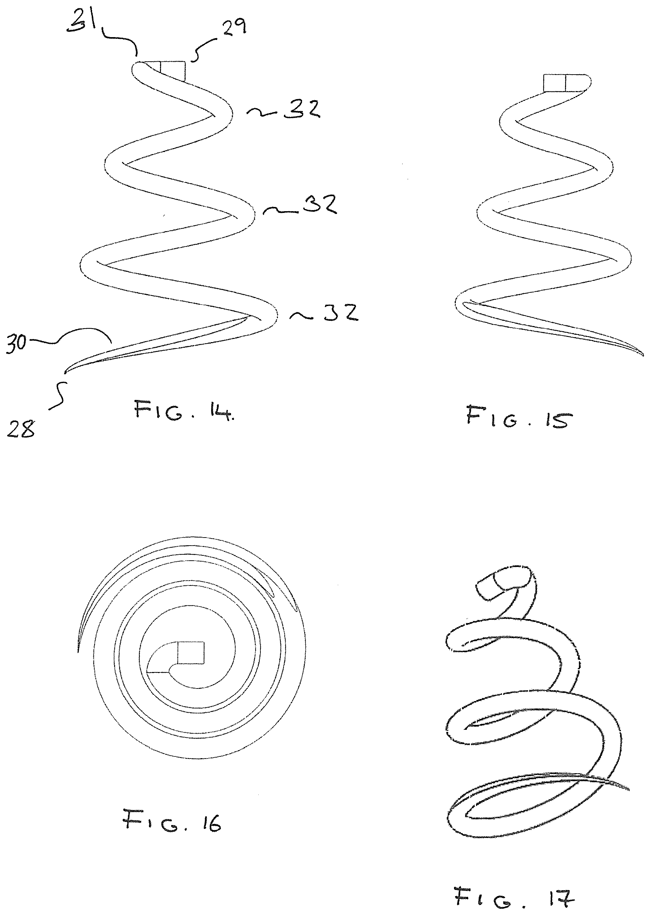

Referring to Figs. 14 to 17 it will be noted that in this case the tapered coil 12 has a pointed tissue insertion tip 28. The coil 12 also has a delivery mechanism interface feature 29.

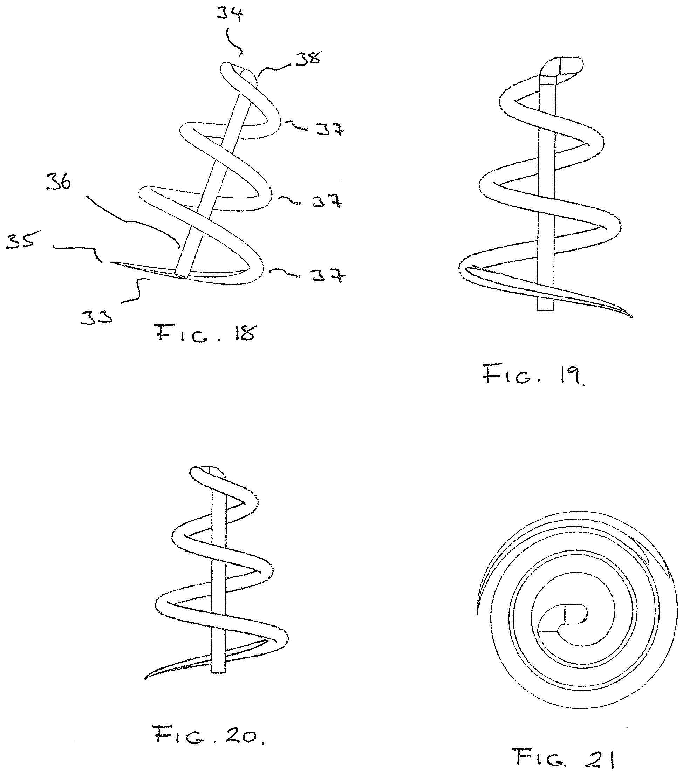

Figs. 18 to 21 illustrate another tapered coil 12 which also has a pointed tissue insertion tip 35 and a delivery mechanism interface feature 38. In this case the coil also has a centering feature 36. The centering feature 36 passes through the centre of the fistula tract and allows attachment of the drainage seton. The centering feature 36 allows centering of the coil in a concentric fashion to the fistula tract internal opening.

It will be appreciated that a coil device with a centering feature such as the centering feature 36 illustrated in Figs. 18 to 21, with or without an attached seton, may be used to close fistula openings that may commonly occur in other areas of the body, such as: biliary (created during gallbladder surgery, connecting bile ducts to the surface of the skin), cervical (either an abnormal opening into the cervix or in the neck), craniosinus (between the space inside the skull and a nasal sinus), enterovaginal (between the bowel and vagina), faecal or anal (the faeces is discharged through an opening other than the anus), gastric (from the stomach to the surface of the skin), metroperitoneal (between the uterus and peritoneal cavity), umbilical (between the navel and gut). These fistulas may be:- blind also known as a sinus (open on one end only, but connects to two structures); complete (has both external and internal openings); horseshoe (connecting the anus to the surface of the skin after going around the rectum); or incomplete (a tube from the skin that is closed on the inside and does not connect to any internal structure).

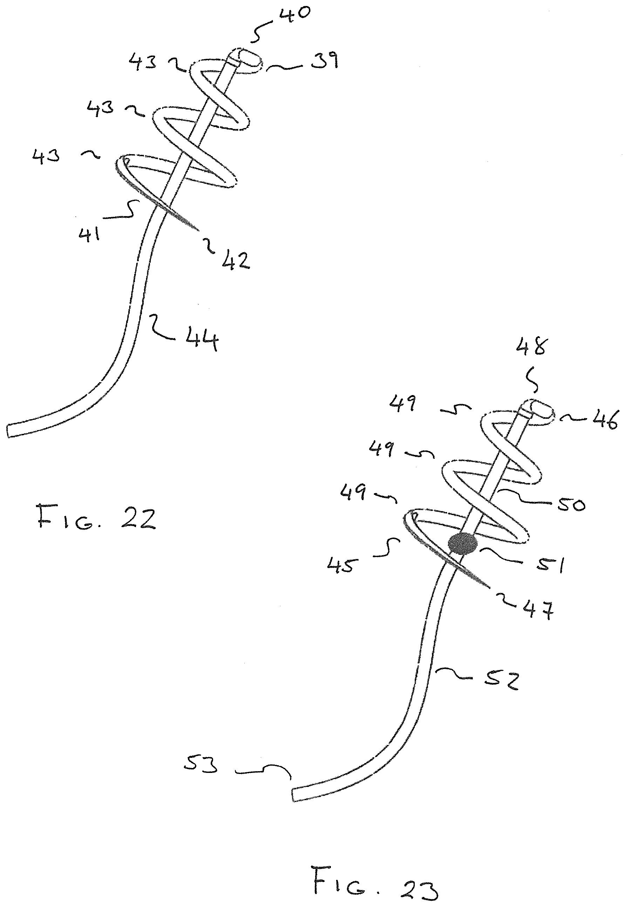

Fig. 22 illustrates a coil device with a centre seton feature 44. In this embodiment the coil, centre feature and drainage seton are constructed from a single continuous monolithic structure.

Fig. 23 illustrates a coil which has a centre feature 50 with a seton attachment feature 51 at the distal end. A drainage seton 52 is attached to the feature 50.

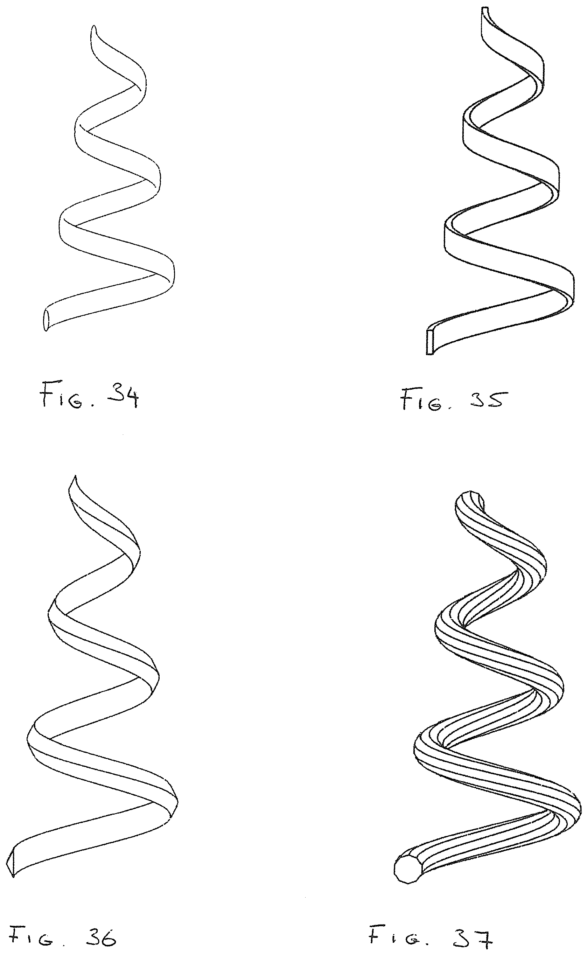

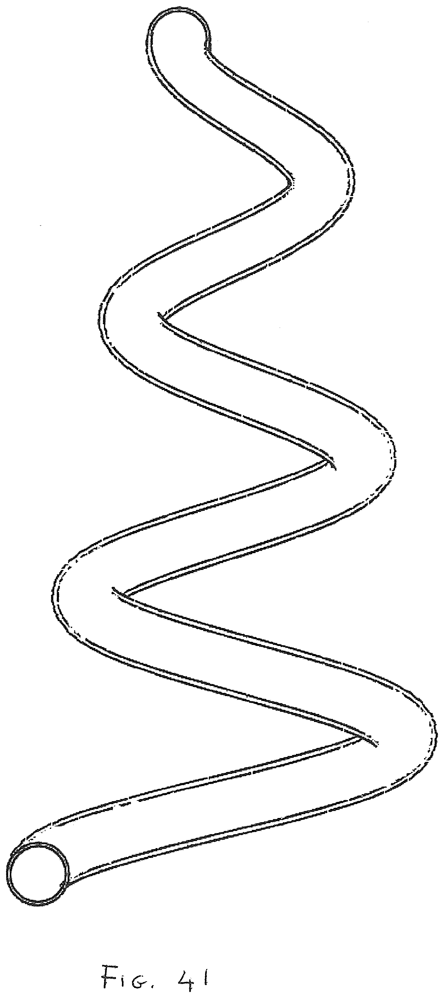

It will be appreciated that the tapered coil may be of any suitable shape in transverse cross section. Some examples are illustrated in Figs. 34 to 44. For example, the coil may be round, oval, triangular, multifaced or ribbon-like. In some cases the coil may be hollow.

The coil may be intended for subsequent removal or may be bioabsorbable.

Typical materials for the coil include
- Bioabsorbable magnesium (including MgFe and other magnesium alloys) would be a material of choice because it offers the strength of stainless steel and similar metals, yet is bioabsorbable. MgFe alloys are well studied and have been used in medical products.
- PLA) and PLGA (poly(lactic-co-glycolic acid)) are bioabsorbable polymers and would be a material of choice as they are commonly used bioabsorbable materials and have been well studied and used in medical products for over 70 years.
- The coil may also be constructed from other common materials used for suture applications

A bioabsorbable tapered coil would be beneficial to treatment of perianal fistulas due to the body's natural tendency to reject foreign materials.

We also describe a delivery device for the perianal fistula treatment device.

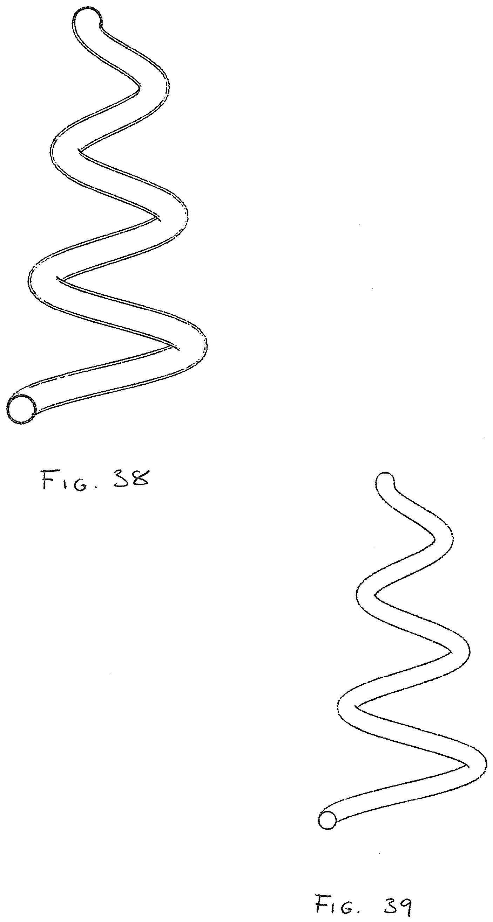

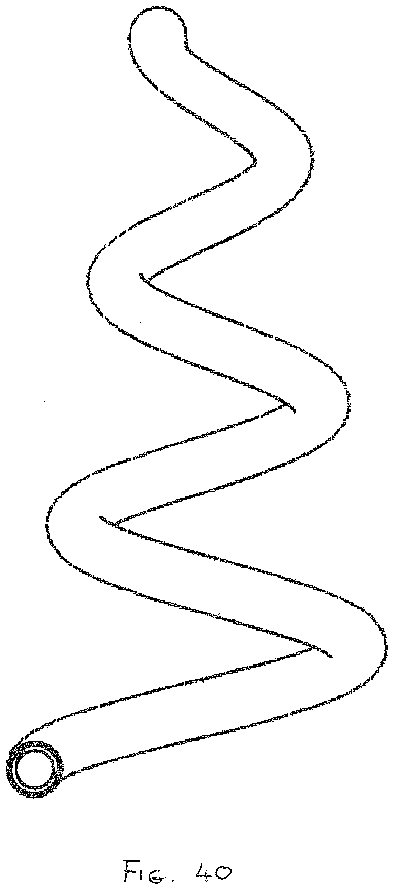

The delivery device may comprise a hollow element such as illustrated in Fig. 38 through which the tapered coil is delivered. The hollow delivery element may comprise a coil. Fig. 39 illustrates a solid coil to be delivered by a hollow delivery element of Fig. 38 and Fig. 40 shows the hollow delivery element with the solid coil in place.

The system consists Fig. 40 of a hollow delivery element Fig. 38 that contains (houses) the implantable element Fig. 39. The implantable element Fig. 39 is housed in the delivery element Fig. 38 during the rotational delivery process. Once the delivery element Fig. 38 has reached the desired delivery position, it is uncoupled from the implantable element Fig. 39 and then rotated in the opposite direction of the direction of delivery (most commonly counter clockwise), leaving the implantable element Fig. 39 in place of the tissue bulk.

It will be appreciated that the system may be reversed with a hollow coil delivered over a solid delivery element.

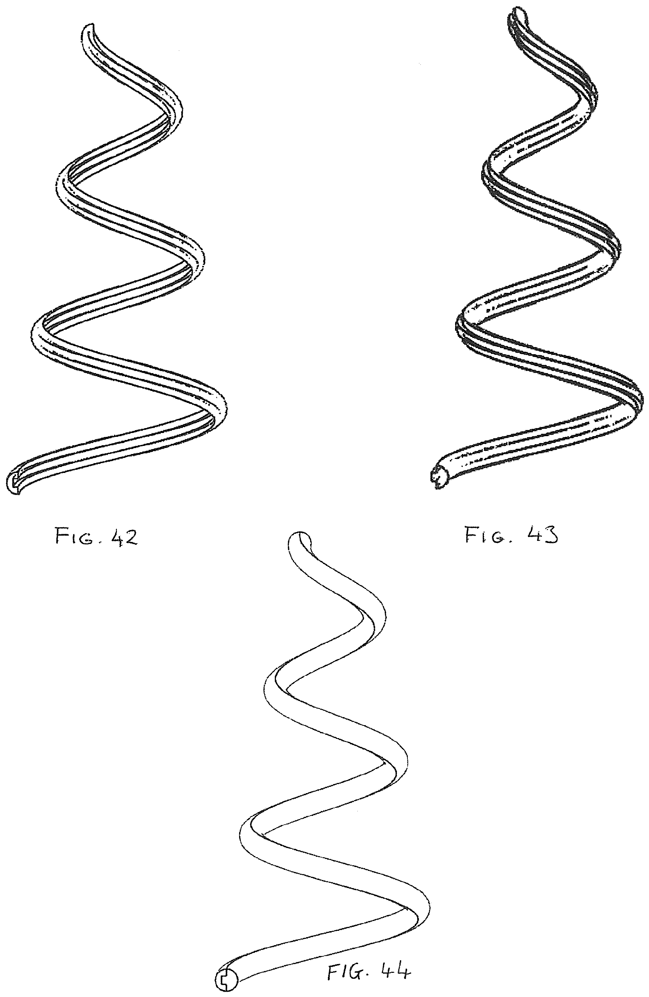

In some cases the delivery device comprises a rail for the tapered coil. The rail and the coil may have interengagable features. Some examples are illustrated in Figs. 42 to 44.

The rail system Fig. 44 consists of an outer implantable coil element Fig. 42, and an inner support rail element Fig. 43. The two elements interlock. The system Fig. 44 is rotated (most commonly in a clockwise direction) to the desired tissue depth. Upon the system Fig. 44 reaching the desired tissue depth, the elements are uncoupled Fig. 42 and the inner support rail is reversed out of the tissue bulk by rotation opposite in direction to insertion (commonly counter clockwise), leaving behind the outer implantable coil element Fig. 42 in place surrounding, compressing and closing the fistula tract.

It will be appreciated that the system may be reversed with an inner implantable coil delivered over an outer support rail.

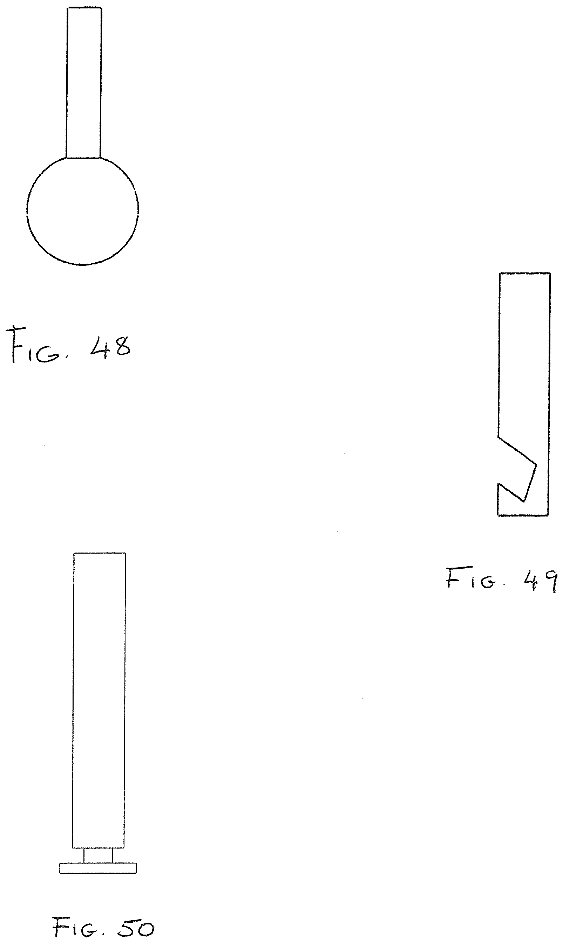

### Ball attachment Figure 48:

The centre feature may have a ball feature along the shaft to aid in anchoring a seton that may be tied or looped around the centre feature. The ball provides a back stop where the knotted or looped seton will not detach from the centre feature.

### Hook attachment Figure 49:

The centre feature may have a hook feature along the shaft to aid in anchoring a seton that may be tied or looped around the centre feature. The hook provides a back stop where the knotted or looped seton will not detach from the centre feature.

### Cleat attachment Figure 50:

The centre feature may have a cleat feature along the shaft to aid in anchoring a seton that may be tied or looped around the waist of the cleat feature. The cleat provides a back stop where the knotted or looped seton will not detach from the centre feature.

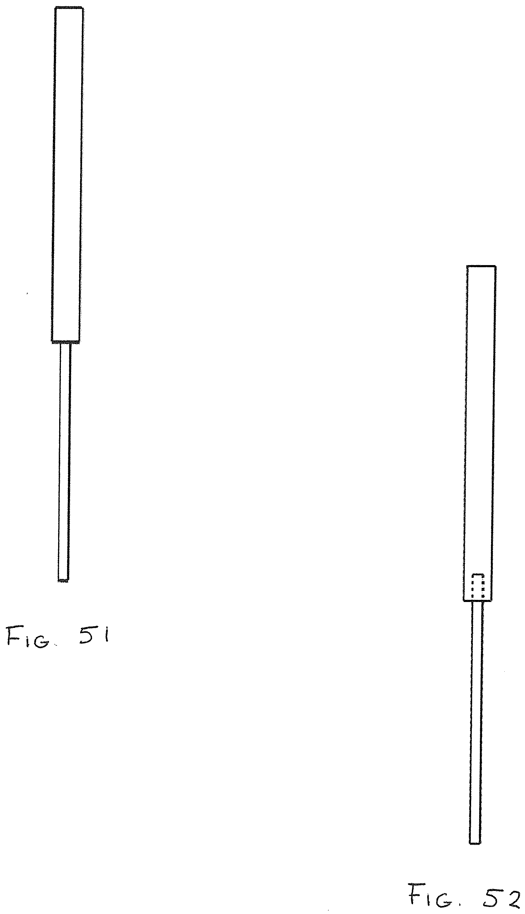

### Internal to Centre feature Figure 51 and 52

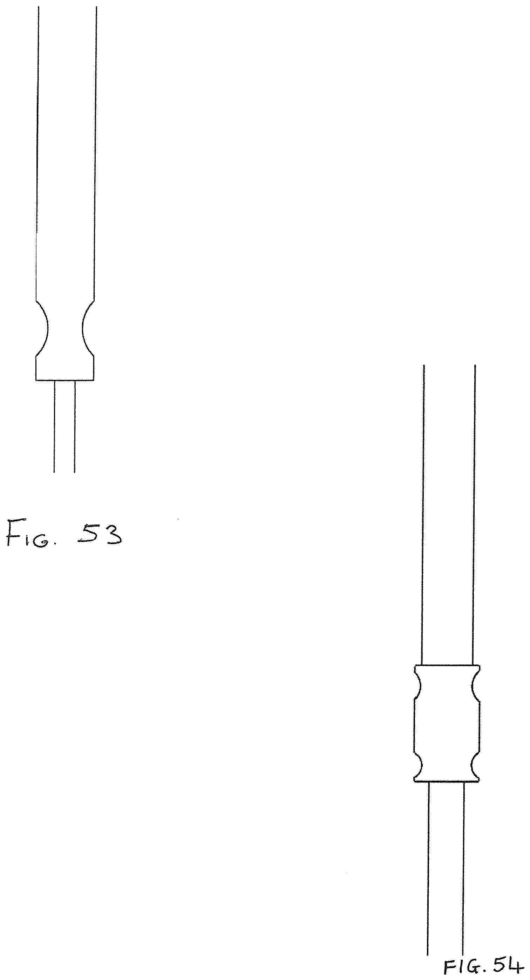

The centre feature may be hollowed as in Figure 51 and the seton may be placed in the hollowed portion. It may be glued or heat staked as shown in Figure 52 or crimped as shown in Fig. 53.

### Butt Joint Figure 54:

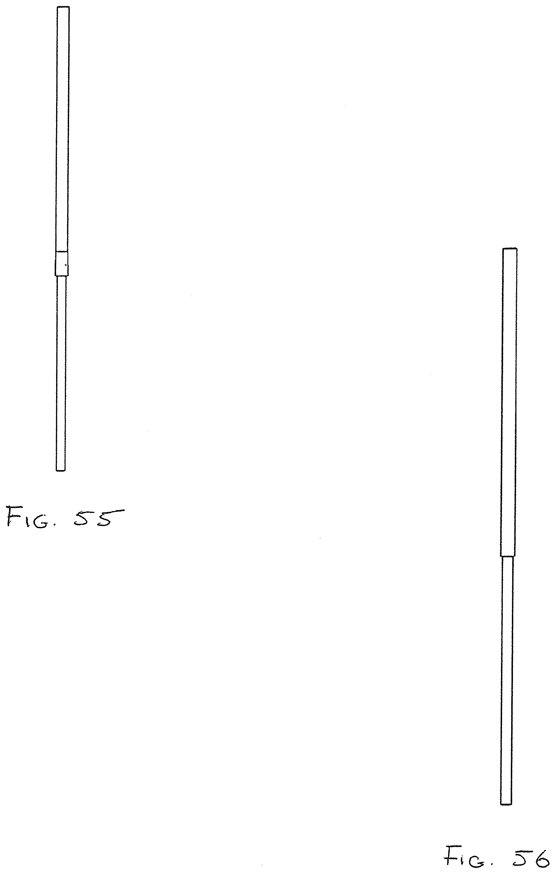

The seton may be attached by a butt joint as shown in Figure 55. The centre feature and seton are inserted into a tubular element with opposite ends facing each other as in Figure 55. The three components may be joined by heating, glue as shown in Fig. 55, or by crimping/swaging as shown in Fig. 54. The join may have multiple crimp points along the tubular element to securely attach the seton and centre feature.

### Thermal bond Figure 56

The seton and centre feature may be thermally bonded / joined together as in Fig. 56, where both materials are made of a material with similar glass transition temperatures (Tg) and brought to their Tg and allowed to flow together creating a secure junction.

It will be appreciated that the embodiments of this system may also incorporate features such as previously described, including, but not limited to, a centre feature, a seton attachment feature, an integrated drainage seton and an integrated sharp tip located on the drive rail.

The seton 12 is used as a guidance and positioning mechanism and once the device is implanted serves as a means of fistula tract drainage. The seton 12 may be constructed of bioabsorbable materials, tissue healing enchantment properties, infection control agents and be constructed of part or composite of these materials.

After the fistula tract preparation, the seton 12 is attached using standard surgical technique to the existing surgical probe, suture, or seton already in place in the fistula tract. Once the seton 12 is attached, the system is pulled through the fistula tract proximally (towards the physician) until the coil device is adjacent to the tissue wall (rectal wall). The seton 12 ensures that the outer leading coil is centred around the outside of the fistula tract. Tension may be applied to the seton 12 as the coil is advanced into the tissue to aid in advancement and to maintain a centred position around the fistula tract.

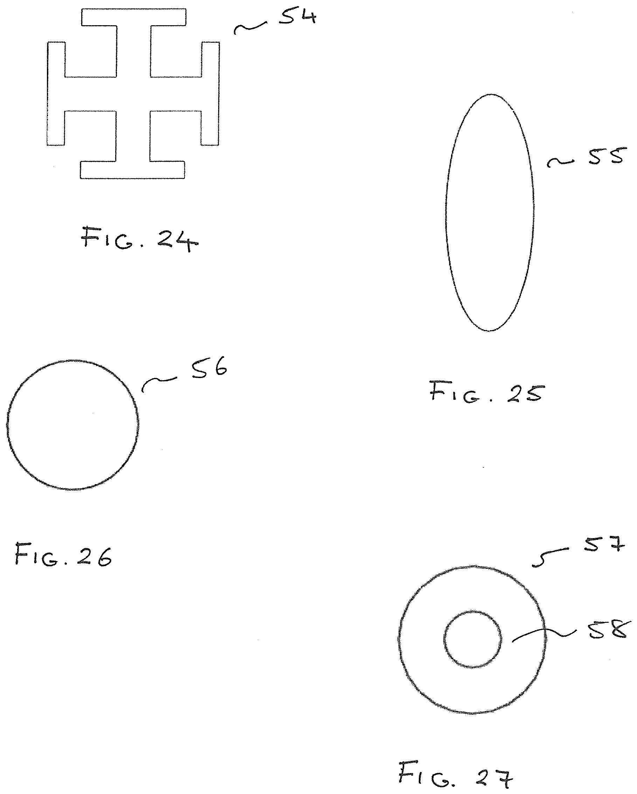

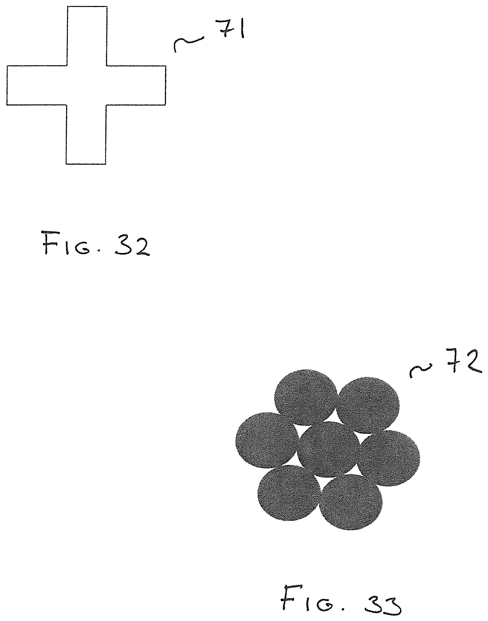

The seton 12 is attached to the central portion of the coil 11. With the coil knitting together the sphincteric muscle and closing the fistula tract's internal opening the seton 12 maintains the proximal portion of the fistula tract's patency to facilitate drainage of any abscess, pus, and new accumulation of bodily fluids to prevent infection occurrence. The seton 12 prevents the tract from closing in on itself proximal of any fluid accumulation and acts as a conduit allowing material drainage between the wall of the tract and the outer wall of the seton 12. The seton 12 may also have a central lumen with tangential drainage holes entering from the external wall of the seton 12. The seton 12 may be constructed with a multi surface external wall to create channels and optimize the fluid drainage and prevent the fistula tract wall from occluding drainage around the seton. The seton 12 may be constructed of part or all elements as described and illustrated in Figs. 24 to 33.

The seton 12 is constructed of materials that are strong enough to allow for surgical placement in the fistula tract. The seton 12 may be constructed of materials that are non-absorbable and meant to be removed at a later time. Alternatively, the seton 12 may be made of materials that bioabsorb throughout and upon completion of the fistula tract healing processes (examples include magnesium, PLA, PLGA). The seton 12 may be constructed of or include anti-infection agents to prevent infection of the fistula tract (silver ions, antibacterial agents). The seton 12 may be constructed of materials that aid in tissue growth (stem cell, collagen matrix). The seton 12 may be constructed of part or all elements as described.

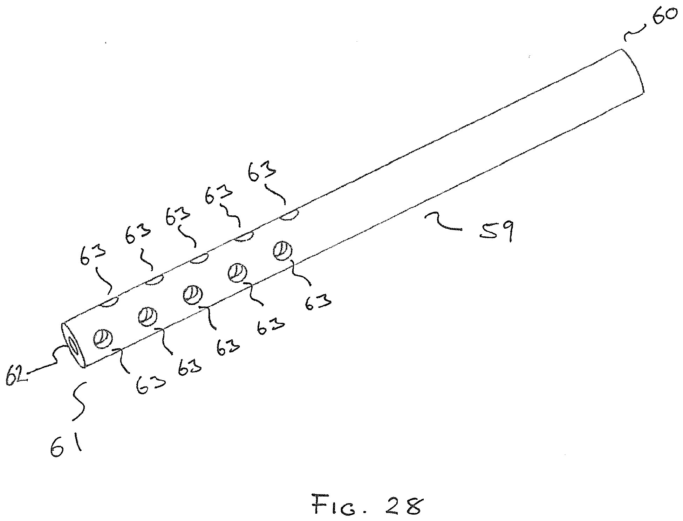

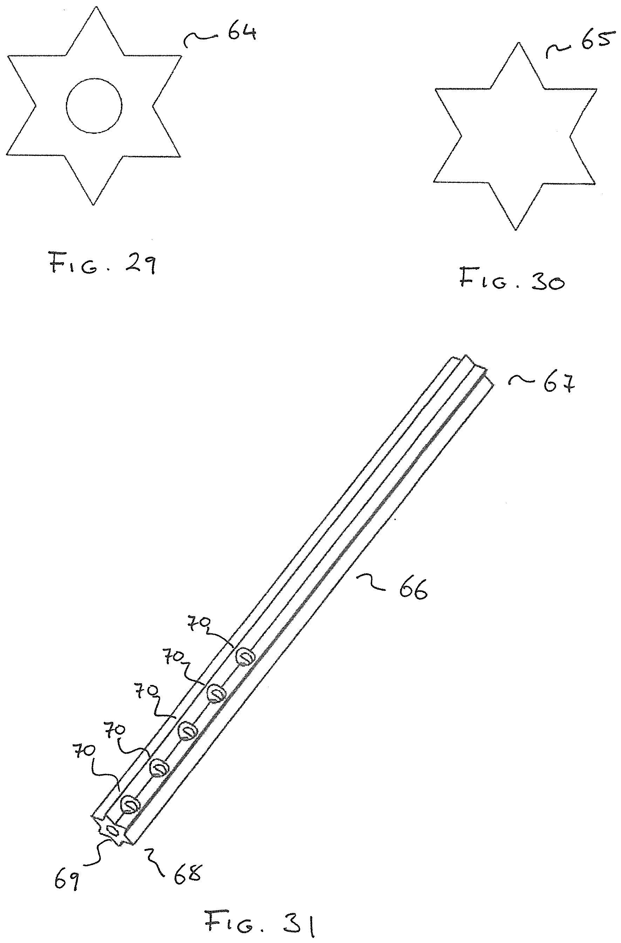

The seton may be of any suitable shape in cross section such as round, oval, cross shape, star or braid as illustrated in Figs. 24 to 26 and Figs. 30, 32, 33. In all cases the seton may be hollow as illustrated in Figs. 27, 28, 29 and 31 to further enhance drainage. The seton may have peripheral holes as illustrated in Figs. 28 and 31 to provide for increased drainage effectivity. The holes allow additional surfaces of drainage, by increasing drainage surface area/channels the fluid drains more quickly and reduces the chance that any of the channels will become occluded and prevent fluid drainage at the same moment in time.

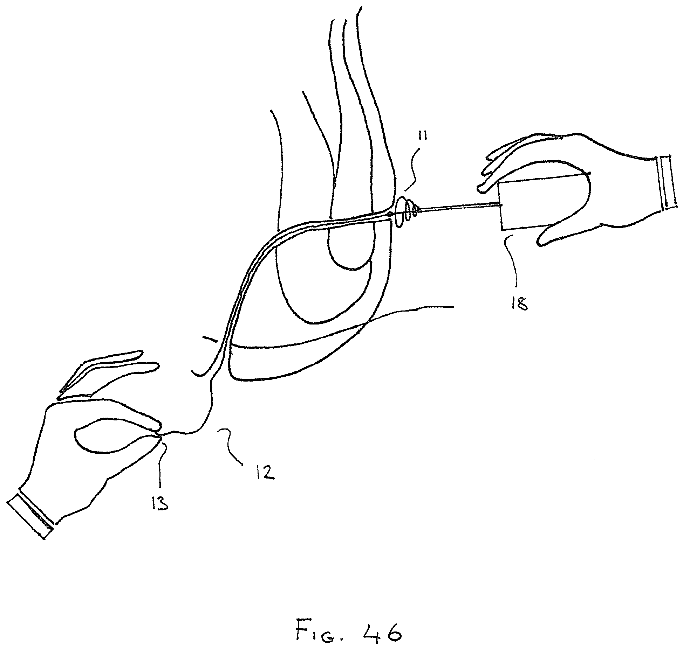

Fig. 46 illustrates the delivery of the system through the fistula tract prior to deployment of the coil. The seton, attached to the centre feature allows the coil to be drawn into apposition against the mucosal wall, and located concentric to the fistula tract internal opening.

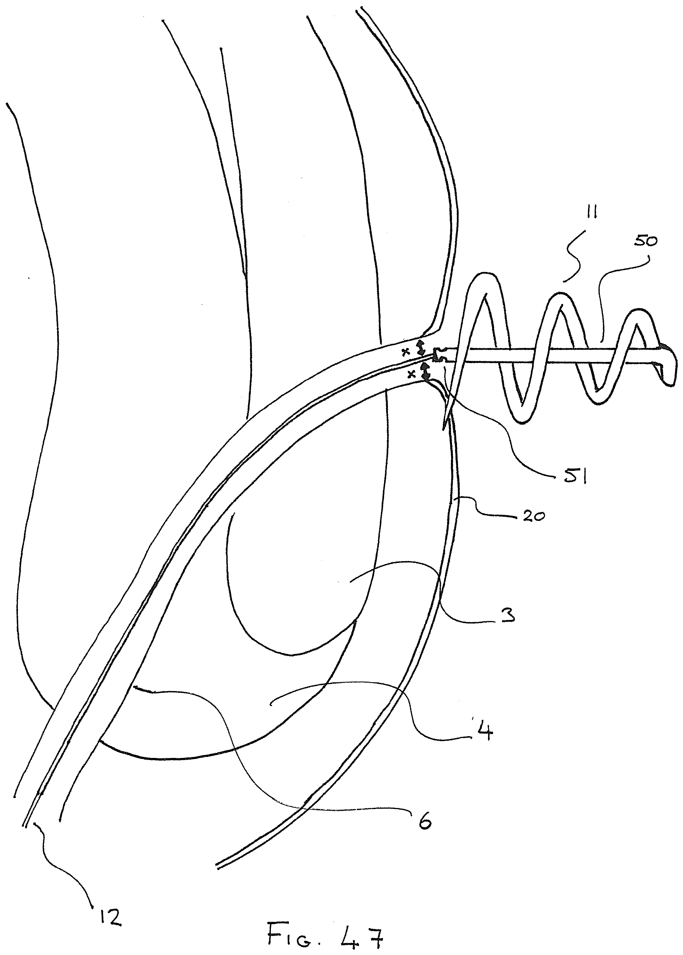

Fig. 47 illustrates the position of the coil prior to deployment and demonstrates the effect of the centering feature in combination with the seton in positioning the coil concentrically around the fistula tract internal opening. (Dimensions 'x' being equal).

As noted above, one or other or both of the coil and seton may comprise bioabsorbable materials.

Typical materials for the coil include:
- Bioabsorbable magnesium (including MgFe and other magnesium alloys) is one material of choice because it offers the strength of stainless steel and similar metals, yet is bioabsorbable. MgFe alloys are well studied and have been used in medical products.

Synthetic bioabsorbable materials may include PLA and PLGA (poly (lactic-co-glycolic acid)) (PLGA, PCL, Polyorthoesters, Poly(dioxanone), Poly(anhydrides), Poly(trimethylene carbonate), Polyphosphazenes), and or natural bioabsorbable materials may include fibrin, collagen, chitosan, gelatin, Hyaluronan are bioabsorbable polymers and would be a material of choice as they are commonly used bioabsorbable materials and have been well studied and used in medical products for over 70 years.

For example, companies such as Ethicon market a number of such products with different absorption rates such as http://www.ethicon.com/healthcare-professionals/products/. Absorbable polymer materials are also available from medical material companies such as Zeus, see http://www.zeusinc.com/advanced-products/absorv-bioabsorbables.

Typical materials for the seton include:
- Bioabsorbable magnesium (including MgFe and other magnesium alloys) is one material of choice because it offers the strength of stainless steel and similar metals, yet is bioabsorbable. MgFe alloys are well studied and have been used in medical products.
- Synthetic bioabsorbable materials may include PLA and PLGA (poly(lactic-co-glycolic acid) ) (PLGA, PCL, Polyorthoesters, Poly(dioxanone), Poly(anhydrides), Poly(trimethylene carbonate), Polyphosphazenes), and or natural bioabsorbable materials may include fibrin, collagen, chitosan, gelatin, Hyaluronan are bioabsorbable polymers and would be a material of choice as they are commonly used bioabsorbable materials and have been well studied and used in medical products for over 70 years.

In one case both the coil and the seton are bioabsorbable, and the seton degrades prior to the degradation of the coil. This may be achieved in a number of different ways, such as the seton being of a different bioabsorbable material to the coil.

For example the coil implant may be constructed of PLLA which degrades slowly, typically within 18 to 36 months depending on formulation, cross section, and surface modifications, and the Seton drain may be constructed of PLGA (85L/15G) which typically degrades "faster" in 1 to 2 months depending on formulation, cross section, and surface modifications

Another method of altering the time of degradation (degradation (absorption) properties) is by providing a reduced cross sectional area, more porosity, less crystallinity, more reactive hydrolytic groups in the backbone, more hydrophilic end groups, and/or more hydrophilic backbone.

In one case, the seton begins to absorb 5 weeks post-surgical implantation. This is variable depending upon the healing time of the patient, with full healing usually occurring within a 5 to 10 week period. By way of example the coil implant may remain for a period of at least 10 weeks after healing and may degrade over a 6 to 18 month time period from the date of implantation.

Advantageously, the closure mechanism of the device is maintained during the entire healing process. In some cases the coil remains in situ to withstand rectal pressures and maintain closure of internal tract opening for at least 10 weeks to prevent re-opening of the tract.

The coil implant may remain in place longer to allow full healing of the internal opening of the fistula tract. The seton drain may degrade at a faster rate compared to the coil implant so long as the seton drain is in place for a long enough time for all remaining abscess and infection, to drain from the fistula tract and any side branches. It is advantageous that the seton drain absorbs faster than the coil so that the patient does not have any visually remnant feature of the device or thoughts of fistula. The seton is not needed for as long a period as the coil implant, with the seton drain absorbing faster than the implant, the patient will not have to return to the surgeon for removal during the internal opening healing process.

Also, the implant remains in place for a long enough period of time (e.g. greater than 1 week) to allow remodelling of the defect in the mucosa and formation of a mucosal layer. This mucosal layer acts as a bacterial seal preventing reinfection of the tract from entering of fasces. The reformation of the musical layer in conjunction with the sphincter muscle closure mechanism prevents fasces entering the tract.

The implant coil and draining seton may be doped or loaded with healing and antimicrobial agents (such as stem cell, silver ions, silver particles, antibiotics, antibacterial agents and the like).

The seton may be of differential bioabsorption wherein the seton is absorbed at a different rate along its length.

The seton may be of differential bioabsorption wherein the distal portion of the drain absorbs more quickly than the proximal portion. This differential absorption of the seton results in the seton remaining attached to the coil via the proximate portion until fully absorbed. Advantageously, this allows for the external opening to close and remove the chance of the seton being pulled out through the external opening.

The seton may also be of differential bioabsorption wherein the proximal portion of the seton absorbs more quickly - in this case the anchoring mechanism of the closure device with relation to the seton could be broken at an earlier time than the full seton absorption allowing the seton to be removed (by the patient or doctor or naturally fall out) through the external opening.

In both differential absorption embodiments, the entire seton would have to remain in place for full healing (and drainage) time of the tract (e.g. 10 weeks).

The bioabsorbable materials used in the construction of the implant coil, or drainage seton, or both, can be both natural or synthetic polymers such as those listed below.

### Natural polymers

▪ Fibrin
▪ Collagen
▪ Chitosan
▪ Gelatin
▪ Hyaluronan

### Synthetic polymers

▪ PLA, PGA, PLGA, PCL, Polyorthoesters
▪ Poly(dioxanone)
▪ Poly(anhydrides)
▪ Poly(trimethylene carbonate)
▪ Polyphosphazenes

The selection of the material used can be made whilst taking the following factors into account.

### Factors that accelerate polymer degradation:

▪ More hydrophilic backbone.
▪ More hydrophilic endgroups.
▪ More reactive hydrolytic groups in the backbone.
▪ Less crystallinity.
▪ More porosity.
▪ Smaller device size.

The implant coil of the system of the invention may be delivered by a number of techniques. In one case the coil is delivered by a coil delivery mechanism. In this case, the implant coil may have an interface region for interfacing with the delivery mechanism.

A perianal fistula treatment device may comprise an implant coil having a tapered portion which is configured for insertion into bulk tissue surrounding a fistula. The implant coil may have a driver interface portion which is configured for engagement with a driver implement for rotation of the coil to draw tissue surrounding a fistula inwardly. The advantages of such an implant coil are:
- Ability to be delivered deep into the sphincter muscle complex allowing for greater anchoring and sphincter muscle apposition at the muscle defect
- Ability to disengage from the delivery mechanism in a spiral nature, allowing reversing of the delivery mechanism through the same tract as delivery preventing further damage to the tissue
- Prevents the mucuosa of the rectum being pulled down towards the sphincter muscle complex
- Ability to be delivered through and past the anoderm resulting in lower pain due to interference with the nerve endings of the anoderm
- Prevention of bacterial tracking by delivering deep sub mucosally allowing a new mucosal lining to form at the fistula internal opening

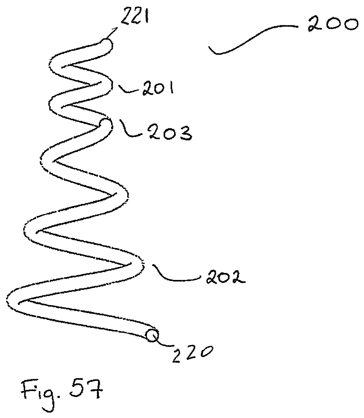

One such implant coil 200 is illustrated in Fig. 57 and comprises a tapered section 202 and a driver interface portion which in this case is provided by straight coil section 201 which has a substantially uniform lateral extent along a length thereof. The implant coil 200 has a leading end 220, a transition region 203 and a trailing end 221. The tapered portion 202 of the coil extends from the leading end 220 to the transition region 203 and the driver interface portion 201 extends from the transition region 203 to the trailing end 221. It will be noted that, as described in other embodiments, the tapered portion of the coil decreases in lateral extent between the leading end 220 and the transition region 203. The leading end 220 may have a pointed tissue insertion tip as illustrated, for example, in Figs. 14 to 17.

The implant is a coiled body structure. The leading end of the implant is the largest coil and initially surrounds the tissue defect with appropriate margin. As the implant is advanced the leading end provides a large surface area to effectively anchor the implant. Each subsequent coil provides (adds to) the anchoring and compression function. The smallest coil towards the trailing end provides the highest amount of tissue compression. As the implant is turned into the tissue each coil further compresses the captured tissue toward the center of the tissue defect, thus effectively completely compressing the surrounding tissue inwardly. The close approximation of tissue allows for the tissue to heal together. This compression provides an effective seal against the pressures generated in the rectum and prevents entering of passing faeces into the fistula tract thus preventing re-infection. The smaller diameters of the implant coils retain the captured tissue from separating and prevents the breakdown of the healing process or foreign material from entering the tissue defect. This is a major advantage over sutures and suture based surgical techniques such as the advancement flap (dermal flap) and the LIFT procedures.

The compression ensures close approximation of tissue throughout the center of the implant. At the most proximal surface the close approximation of tissue provides support to the healing mucosal lining of the rectum over the implant and tissue defect. Thus the healing tissue is fully supported by the implant during the healing process and is capable of surviving pressures of 150mmHg and upwards of 200mmHg which can be generated in the rectum.

The coil is delivered submucosal (at a predetermined depth) below the surface of the mucosa. This ensures that there is a full mucosal seal at the rectal mucosa surface to provide for a bacterial seal barrier. With the implant just below the surface the tissue is drawn inwards for complete compression and supports the mucosa healing process.

As the implant is turned into the tissue the compression becomes greater along the depth of the coil (progressive compression) and the length of the tract captured internal of the implant is compressed completely. This close approximation of tissue aids in the healing process.

In this and other embodiments the implant body is in the form of an open tapered coil body (for example, without a cross bar or other centering feature) in which the leading edge, (into the muscle) is of a larger diameter than the trailing edge, (rectum surface). The trailing portion is of smaller diameter than the leading portion. The coil is of open form, therefore there is no inward protrusion at either the proximal nor distal end of the body. This open form factor enables the implant to be driven into the tissue body to a pre-determined depth (depending on the taper) which results in progressive tissue compression.

At least the driver interface portion of the implant coil is solid and in some cases all of the implant coil is solid. Alternatively, as described above and below, the implant coil or at least part thereof may be hollow.

In this embodiment, preferably the treatment device also includes a seton of the type described above. In some cases the seton is not mounted or attached to the implant coil during delivery but may be attached so as to extend from the coil when the coil is in situ. In some cases the seton may be embedded in the sphincter muscle complex and lead so that the end of the seton protrudes through the external opening of the fistula tract.

The implant is delivered using any suitable delivery device as described. **In** one case the delivery device comprises a driver implement which interfaces with the implant coil and is used to rotate the coil to draw tissue surrounding a fistula inwardly. The driver implement preferably interfaces with the driver interface of the implant coil.

In some cases the driver implement comprises a driver coil which is configured for engagement with the driver interface of the implant coil. The driver coil may have a substantially uniform lateral extent along a length thereof for engagement with the corresponding driver interface portion of the implant coil.

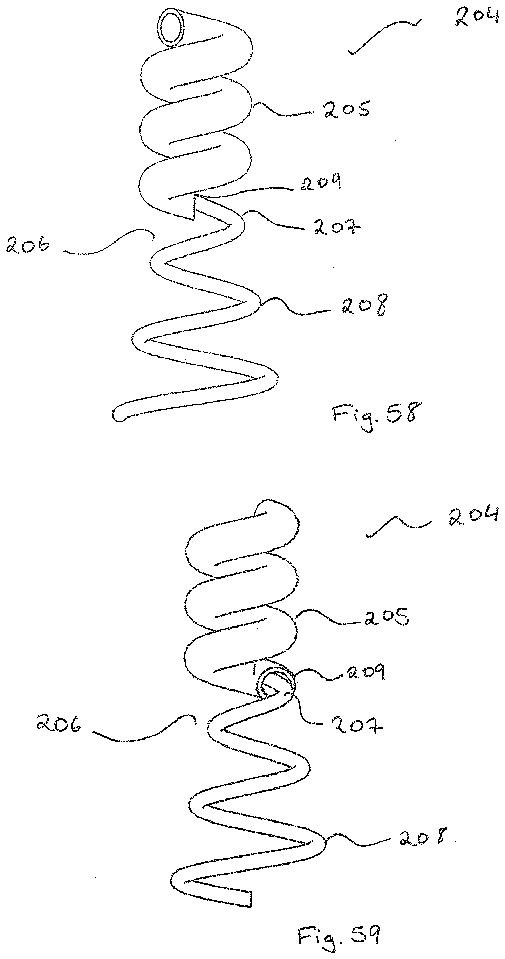

In one embodiment as illustrated in Figs. 58 and 59 a driver coil 205 is hollow for interfacing with the interface portion 201 of the implant coil.

Figs. 58 and 59 illustrate an assembly 204 in which a solid implant coil 206 is delivered by a straight hollow delivery coil 205. The coil implant 206 has a straight profile section 207 and a tapered section 208. The straight profile section 207 fits within the internal channel of the delivery coil 205 up to the interface 209, whereby the hollow coil 205 is rotated which in turn rotates the implant coil 206 for delivery.

The hollow sections of the delivery coil in all cases may comprise a single turn, multiple turns or part thereof or the entire construct.

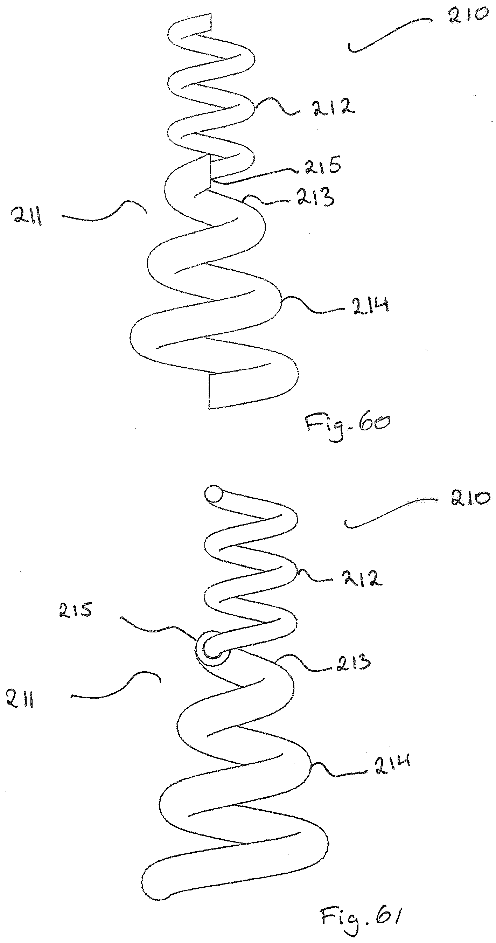

Figs. 60 and 61 illustrate an assembly 210 in which a hollow implant coil 211 is delivered by a straight solid delivery coil 212. The coil implant 211 has a straight profile section 213 and a tapered section 214. The straight solid delivery coil 212 fits within the internal channel of the hollow coil implant 211 up to the interface 215, whereby the solid delivery coil 212 is rotated which in turn rotates the hollow coil implant 211 for delivery.

The straight sections of the coil in all cases may comprise a single turn, multiple turns or part thereof.

The hollow sections of the implant coil in all cases may comprise a single turn, multiple turns or part thereof or the entire construct.

The delivery system has the following advantages:
- Ability to follow the tract of the implant coil allowing deep delivery to the sphincter muscle complex allowing for greater anchoring and sphincter muscle apposition at the muscle defect
- Ability to disengage and retract in a spiral nature, reversing through the same tract as delivery preventing further damage to the tissue
- Prevents the mucuosa of the rectum being pulled down towards the sphincter muscle complex
- Enables the implant to be delivered through and past the anoderm resulting in lower pain due to interference with the nerve endings of the anoderm
- Prevention of bacterial tracking by delivering deep sub mucosally

These delivery mechanisms may be coupled to a manually operated, trigger operated user interface or similar.

In current techniques for treating a fistula a surgeon identifies the external opening of the fistula tract and carefully inserts a probe through the external opening, through the fistula tract and through the internal opening of the fistula. The probe is then extended back through the rectum and a localisation seton or suture is attached to the end of the probe which is then drawn back through the rectum and the fistula tract until it exits through the external opening of the fistula tract. The localisation seton loop is then tied off.

The implant and delivery system of the invention is compatible with this known current technique. In the invention the probe or the localisation seton may be used to guide the leading end of the implant coil and/or the drainage seton.

Modifications and additions can be made to the embodiments of the invention described herein without departing from the scope of the invention, within the scope of the claims. For example, while the embodiments described herein refer to particular features, the invention includes embodiments having different combinations of features. The invention also includes embodiments that do not include all of the specific features described.

The invention is not limited to the embodiments hereinbefore described, which may be varied in construction and detail within the scope of the claims.

## Claims

1. A perianal fistula treatment system (204) comprising:
a perianal fistula treatment device comprising an implant coil (200, 211) having a tapered portion (202, 208) which is configured for insertion into bulk tissue surrounding a fistula, and a driver interface portion (201, 207, 213) which is configured for engagement with a driver for rotation of the coil to draw tissue surrounding a fistula inwardly, wherein the driver interface portion of the implant coil has a substantially uniform lateral extent along a length thereof; and
a driver implement (205, 212) for rotation of the implant coil to draw tissue surrounding a fistula inwardly, said perianal fistula treatment system being **characterized in that** said driver implement comprises a driver coil (205, 212) which is configured for engagement with the driver interface of the implant coil, and
the driver coil (205, 212) has a substantially uniform lateral extent along a length thereof for engagement with the corresponding driver interface portion of the implant coil.

2. A perianal fistula treatment system as claimed in claim 1, wherein at least a portion of the implant coil (200) is bioabsorbable.

3. A perianal fistula treatment system as claimed in either of claims 1 or 2 further comprising a drainage seton (12) extending from the implant coil.

4. A perianal fistula treatment system as claimed in any preceding claim, wherein the implant coil has a leading end (220), a transition region (203), and a trailing end (221), the tapered portion (202) of the implant coil extending from the leading end to the transition region and the driver interface portion extending from the transition region (203).

5. A perianal fistula treatment system as claimed in claim 4, wherein the tapered portion (202) of the implant coil decreases in lateral extent between the leading end and the transition region.

6. A perianal fistula treatment system as claimed in claim 5, wherein the leading end (220) of the implant coil tapered portion includes a pointed tissue insertion tip.

7. A perianal fistula treatment system as claimed in any preceding claim, wherein at least the driver interface portion (207) of the implant coil is solid.

8. A perianal fistula treatment system as claimed in any preceding claim, wherein the implant coil (200) is solid.

9. A perianal fistula treatment system as claimed in any preceding claim, wherein the shape of the implant coil in cross section is selected from one or more of round, oval, triangular, multifaced and ribbon.

10. A perianal fistula treatment system as claimed in any of claims 3 to 9, wherein at least a portion of the seton (12) is bioabsorbable.

11. A perianal fistula treatment system as claimed in claim 10, wherein the seton (12) is of differential bioabsorption, and a proximal portion of the seton is bioabsorbable.

12. A perianal fistula treatment system as claimed in claim 11, wherein at least a portion of the implant coil (200) is bioabsorbable and at least a portion of the seton is configured to bioabsorb in advance of bioabsorption of the implant coil.

## Patentansprüche

1. System (204) zur Behandlung von perianalen Fisteln, das Folgendes umfasst:
eine Vorrichtung zur Behandlung von perianalen Fisteln, die Folgendes umfasst: eine Implantatspirale (200, 211), die Folgendes aufweist: einen konischen Teil (202, 208), der für die Einführung in die Masse des Gewebes, das eine Fistel umgibt, konfiguriert ist, und einen Antriebsanschlussstellenteil (201, 207, 213), der für das Eingreifen in einen Antrieb zur Rotation der Spirale konfiguriert ist, um Gewebe, das eine Fistel umgibt, nach innen zu ziehen, wobei der Antriebsanschlussstellenteil der Implantatspirale entlang einer Länge davon eine im Wesentlichen gleichmäßige laterale Ausdehnung aufweist; und
ein Antriebsinstrument (205, 212) zur Rotation der Implantatspirale, um Gewebe, das eine Fistel umgibt, nach innen zu ziehen, wobei das System zur Behandlung von perianalen Fisteln **dadurch gekennzeichnet ist, dass** das Antriebsinstrument Folgendes umfasst:
eine Antriebsspirale (205, 212), die zum Eingreifen in die Antriebsanschlussstelle der Implantatspirale konfiguriert ist, und
wobei die Antriebsspirale (205, 212) entlang einer Länge davon eine im Wesentlichen gleichmäßige laterale Ausdehnung zum Eingreifen in den entsprechenden Antriebsanschlussstellenteil der Implantatspirale aufweist.

2. System zur Behandlung von perianalen Fisteln nach Anspruch 1, wobei zumindest ein Teil der Implantatspirale (200) biologisch absorbierbar ist.

3. System zur Behandlung von perianalen Fisteln nach einem der Ansprüche 1 oder 2, das weiter eine Drainagefadeneinlage (12) umfasst, die sich von der Implantatspirale erstreckt.

4. System zur Behandlung von perianalen Fisteln nach einem vorstehenden Anspruch, wobei die Implantatspirale ein führendes Ende (220), einen Übergangsbereich (203) und ein hinteres Ende (221) aufweist, wobei sich der konische Teil (202) der Implantatspirale von dem führenden Ende zu dem Übergangsbereich erstreckt und sich der Antriebsanschlussstellenteil von dem Übergangsbereich (203) erstreckt.

5. System zur Behandlung von perianalen Fisteln nach Anspruch 4, wobei der konische Teil (202) der Implantatspirale in lateraler Ausdehnung zwischen dem führenden Ende und dem Übergangsbereich abnimmt.

6. System zur Behandlung von perianalen Fisteln nach Anspruch 5, wobei das führende Ende (220) des konischen Teils der Implantatspirale eine spitze Gewebeeinführspitze einschließt.

7. System zur Behandlung von perianalen Fisteln nach einem vorstehenden Anspruch, wobei zumindest der Antriebsanschlussstellenteil (207) der Implantatspirale massiv ist.

8. System zur Behandlung von perianalen Fisteln nach einem vorstehenden Anspruch, wobei die Implantatspirale (200) massiv ist.

9. System zur Behandlung von perianalen Fisteln nach einem vorstehenden Anspruch, wobei die Form der Implantatspirale im Querschnitt aus einer oder mehreren von rund, oval, dreieckig, mehrseitig und Band ausgewählt ist.

10. System zur Behandlung von perianalen Fisteln nach einem der Ansprüche 3 bis 9, wobei zumindest ein Teil der Fadeneinlage (12) biologisch absorbierbar ist.

11. System zur Behandlung von perianalen Fisteln nach Anspruch 10, wobei die Fadeneinlage (12) von unterschiedlicher Bioabsorption ist und ein proximaler Teil der Fadeneinlage biologisch absorbierbar ist.

12. System zur Behandlung von perianalen Fisteln nach Anspruch 11, wobei zumindest ein Teil der Implantatspirale (200) biologisch absorbierbar ist und zumindest ein Teil der Fadeneinlage zur Bioabsorption im Voraus der Bioabsorption der Implantatspirale konfiguriert ist.

## Revendications

1. Système de traitement de fistule périanale (204) comprenant :
un dispositif de traitement de fistule périanale comprenant une bobine d'implant (200, 211) ayant une partie effilée (202, 208) qui est configurée pour une insertion dans la masse de tissu entourant une fistule, et une partie d'interface de commande (201, 207, 213) qui est configurée pour entrer en prise avec un dispositif de commande pour la rotation de la bobine pour tirer le tissu entourant une fistule vers l'intérieur, dans lequel la partie d'interface de commande de la bobine d'implant a une étendue latérale substantiellement uniforme le long d'une longueur de celle-ci ; et
un instrument de commande (205, 212) pour la rotation de la bobine d'implant pour tirer le tissu entourant une fistule vers l'intérieur, ledit système de traitement de fistule périanale étant **caractérisé en ce que** ledit instrument de commande comprend une bobine de commande (205, 212) qui est configurée pour entrer en prise avec l'interface de commande de la bobine d'implant, et la bobine de commande (205, 212) a une étendue latérale substantiellement uniforme le long d'une longueur de celle-ci pour entrer en prise avec la partie d'interface de commande correspondante de la bobine d'implant.

2. Système de traitement de fistule périanale selon la revendication 1, dans lequel au moins une partie de la bobine d'implant (200) est bio-absorbable.

3. Système de traitement de fistule périanale selon l'une des revendications 1 ou 2 comprenant en outre un séton de drainage (12) s'étendant depuis la bobine d'implant.

4. Système de traitement de fistule périanale selon l'une quelconque des revendications précédentes, dans lequel la bobine d'implant a une extrémité d'attaque (220), une région de transition (203), et une extrémité de fuite (221), la partie effilée (202) de la bobine d'implant s'étendant de l'extrémité d'attaque à la région de transition et la partie d'interface de commande s'étendant depuis la région de transition (203).

5. Système de traitement de fistule périanale selon la revendication 4, dans lequel la partie effilée (202) de la bobine d'implant diminue en étendue latérale entre l'extrémité d'attaque et la région de transition.

6. Système de traitement de fistule périanale selon la revendication 5, dans lequel l'extrémité d'attaque (220) de la partie effilée de bobine d'implant inclut un bout pointu d'insertion dans le tissu.

7. Système de traitement de fistule périanale selon l'une quelconque des revendications précédentes, dans lequel au moins la partie d'interface de commande (207) de la bobine d'implant est pleine.

8. Système de traitement de fistule périanale selon l'une quelconque des revendications précédentes, dans lequel la bobine d'implant (200) est pleine.

9. Système de traitement de fistule périanale selon l'une quelconque des revendications précédentes, dans lequel la forme de la bobine d'implant en coupe droite est sélectionnée parmi une ou plusieurs des formes suivantes : ronde, ovale, triangulaire, multiface et de ruban.

10. Système de traitement de fistule périanale selon l'une quelconque des revendications 3 à 9, dans lequel au moins une partie du séton (12) est bio-absorbable.

11. Système de traitement de fistule périanale selon la revendication 10, dans lequel le séton (12) est de bio-absorption différentielle, et une partie proximale du séton est bio-absorbable.

12. Système de traitement de fistule périanale selon la revendication 11, dans lequel au moins une partie de la bobine d'implant (200) est bio-absorbable et au moins une partie du séton est configurée pour être bio-absorbé avant la bio-absorption de la bobine d'implant.
